# EUROPEAN PATENT SPECIFICATION

(11) **EP 1 476 160 B1**
(45) Date of publication and mention of the grant of the patent: **15.10.2008**
(21) Application number: 02711864.5
(22) Date of filing: 13.02.2002
(51) Int. Cl.: A61K 31/49, A61K 31/35, A61P 33/06

(54) **PHARMACEUTICAL COMBINATION OF ARTESUNATE AND MEFLOQUINE FOR THERAPY OF MALARIA**
ZUSAMMENSETZUNG VON ARTENUSAT UND MEFLOQUIN ZUR BEHANDLUNG VON MALARIA
FORMULATION PHARMACEUTIQUE ASSOCIANT DE L'ARTESUNATE ET DE LA MEFLOQUINE POUR TRAITER LE PALUDISME

(43) Date of publication of application: 17.11.2004
(73) Proprietor: Mepha Ltd., 4157 Aesch (CH)
(72) Inventor: MÜLLER, Edgar, 79189 Bad Krozingen (DE); SCHEIWE, Max, Werner, 79689 Maulburg (DE)
(74) Representative: Braun, André jr.
(86) International application number: PCT/EP2002/001500
(87) International publication number: WO 2003/075927

(56) References cited:
- EP-A- 0 362 810
- PRICE R N ET AL: "Artesunate / mefloquine treatment of multi-drug resistant falciparum malaria." TRANSACTIONS OF THE ROYAL SOCIETY OF TROPICAL MEDICINE AND HYGIENE, (1997 SEP-OCT) 91 (5) 574-7. , XP008009362
- WILAIRATANA P ET AL: "A clinical trial of combination of artesunate and mefloquine in the treatment of acute uncomplicated falciparum malaria: a short and practical regimen." SOUTHEAST ASIAN JOURNAL OF TROPICAL MEDICINE AND PUBLIC HEALTH, (1998 DEC 29 (4) 696-701. , XP008009363
- MCGREADY R ET AL: "Randomized comparison of mefloquine - artesunate versus quinine in the treatment of multidrug-resistant falciparum malaria in pregnancy." TRANSACTIONS OF THE ROYAL SOCIETY OF TROPICAL MEDICINE AND HYGIENE, (2000 NOV-DEC) 94 (6) 689-93. , XP008009186
- NOSTEN F ET AL: "Effects of artesunate - mefloquine combination on incidence of Plasmodium falciparum malaria and mefloquine resistance in western Thailand: a prospective study." LANCET, (2000 JUL 22) 356 (9226) 297-302. , XP004263762
- SHWE T ET AL: "Influence of blister packaging on the efficacy of artesunate + mefloquine over artesunate alone in community-based treatment of non-severe falciparum malaria in Myanmar." BULLETIN OF THE WORLD HEALTH ORGANIZATION, (1998) 76 SUPPL 1 35-41. , XP008009180

## Description

The present invention relates to pharmaceutical combinations of artesunate and mefloquine which have antimalarial activity. More particularly, the invention is concerned with the effective amounts of said combinations and their use in the treatment of malaria in a 3-day course.

Malaria is considered to be the most important of the human parasitic diseases, as it causes about 300 to 500 million clinical cases and 1.5 to 2.7 million deaths per year. Despite massive international attempts to eradicate malaria, it remains a serious endemic disease in many areas of Africa, Asia, Latin America and Oceania. One of the major factors contributing to the continued presence of malaria is the emergence of malaria parasites that are resistant to one or more antimalarial compounds.

Human malaria is caused by four species of the protozoa *Plasmodium: Plasmodium falciparum, Plasmodium malariae, Plasmodium vivax and Plasmodium ovale,* with *Plasmodium falciparum* being the most common and virulent. The parasite is transmitted to humans by the bite of Anopheles mosquitoes.

Artemisinin and its derivatives, such as artesunate, are the most rapidly acting antimalarial drugs and are effective against *falciparum* malaria including multi-drug resistant infections. They are rapidly effective and produce faster resolution of parasitemia and fever than all other compounds. The duration of antimalarial activity is short. However, when these drugs are used alone, particularly for courses of only 3-5 days, recrudescence rates are high. Despite their excellent intrinsic antimalarial activity, drug exposure to the infective parasite population for only two or three life cycles is insufficient to eradicate all the malaria parasites in the body.

To improve efficacy and delay the onset of resistance, these drugs are often used in combination with another effective antimalarial, such as mefloquine, which is eliminated more slowly with a terminal elimination half-life of 2-3 weeks. This combination regimen gives higher overall cure rates, and importantly, decreases the risk of high-grade resistance, i.e., a potentially lethal failure to respond to treatment.

Nosten et al investigated in two parallel studies the optimal dosage regimen for multidrug-resistant *falciparum* malaria with an artesunate-mefloquine combination (The Journal of Infectious Diseases 1994;170: 971-7).

In both studies all patients received mefloquine, 25 mg (base/kg). Study 1 compared mefloquine alone with mefloquine plus a *single-dose* (4 mg/kg) artesunate at the same time. Study 2 compared mefloquine alone with 3 days of artesunate plus mefloquine given on the second day, not on the first. The administered artesunate was 10 mg (total)/kg beginning with 4 mg/kg on the first day followed by smaller doses either once daily or twice.

Nosten et al found that the frequency of early vomiting after mefloquine (within 1 hour) was reduced by half when the mefloquine was given not before day 2 of treatment.

To optimize mefloquine administration in an artesunate-mefloquine combination, Price et al compared a 3-day treatment of acute uncomplicated *falciparum* malaria with artesunate followed by mefloquine on different days in a single-dose or in splitted doses with or without food. (Antimicrobial Agents and Chemotherapy, Feb. 1999, p. 341-46).

All patients received artesunate (4 mg/kg/day) for 3 days and mefloquine, either as a splitted dose, or as a single-dose (25 mg/kg) on day 0 or on day 2. Price et al concluded that acute malaria has to be associated with reduced absorption of oral mefloquine, and vomiting is also more likely if mefloquine is administered in the acute phase. On the ground of these results they suggested that mefloquine should be administered during recovery, that means it should be delayed until the second or third day after the first dose of artesunate. Further, they also found that there were no significant differences between patients receiving mefloquine in split- or single-doses.

Thus, the teachings of the prior art are related to artesunate-mefloquine co-administrations that improve the dosage regimen and emphasise the delayed introduction of mefloquine. They do not address the problems that the present invention aims to solve, namely, an artesunate-mefloquine combination for malaria therapy that is not only effective and safe but also simple in administration resulting in a maximum of patients' compliance.

Combination therapy has been found to be vulnerable, if the strategy requires several drugs to be taken at different days and times. In addition, the rapid alleviation of symptoms due to the effect of artesunate often leads to abrupt end of the therapy as the patient feels well. This leads to high risk of recurrence of the disease and the development of drug resistance due to an insufficient treatment.

Compliance of the patient is, therefore, a crucial consideration and a key determinant in the successful treatment of malaria regarding both efficacy of the therapy and prevention of drug resistance. Long duration regimens, complex dosing schemes, poor understanding of the patient of how or why to adhere to the recommended regimen as well as rapid perception of wellness induce the patient to discontinue the therapy prematurely.

Therefore, it is a further need to provide antimalarial combinations which allow a malaria therapy that is not only effective, well-tolerated, prevents recrudescence and the emergence of clinical resistance, but also improves patients' compliance. The latter is achieved best if the treatment is simple and of short duration.

Suprisingly, a combination of artesunate and mefloquine has now been found that provides a simple fixed-dose treatment administered once a day for 3 days. The combination is unexpectedly well tolerated with a low rate of side-effects, especially of vomiting, and of equally high cure-rates than in comparable treatments. Due to the simple dosing regimen the patients' compliance is excellent.

The term "fixed-dose" is used herein meaning that the daily dosage of both antimalarials are the same every day for a given treatment course. The amount of the effective dose is individually determined by the medical practicioner as described below.

The present invention, therefore, relates to a pharmaceutical combination which consists of an effective amount of artesunate and mefloquine. The combination is used for the treatment of malaria.

Thus, a method for the treatment of malaria in a 3-day course is also provided administering an effective amount of the combination of artesunate and mefloquine wherein the dosage of artesunate and the dosage of mefloquine is each day the same, and it is preferably administered once a day simultaneously.

Simultaneous dosing of both drugs, when mentioned in this invention, is defined as once daily co-administration of artesunate and mefloquine from the first day of treatment on, and for a total of 3 days.

The amount of the combination of artesunate and mefloquine required to be effective as an antimalarial will, of course, vary and is ultimately at the discretion of the medical practitioner. The factors to be considered include the nature and severity of the malaria to be treated, route of administration, high or low transmission area, the immune status of the patient as well as age and weight of the patient.

In case the patient has never been exposed to malaria infection before, or if he is living in an area with low transmission of the disease, e.g. Asia, a higher mefloquine dosage regimen will be appropriate than in areas of high transmission, where partial immunity can be expected, such as in Africa.

The artesunate-mefloquine combination of the present invention is particularly recommended to treat *falciparum* malaria parasites which are resistant to other antimalarial drugs, or malaria caused by mixed malaria parasites.

In general, a suitable effective dose of artesunate is 20-700 mg per day, preferably 50-550 mg per day and most preferably 200 mg per day. The amount of mefloquine is advantageously 100-900 mg per day, preferably 150-750 mg per day and most preferably 250-500 mg per day.

In a very preferred embodiment of the invention treating acute uncomplicated *P*. *falciparum* malaria, the effective dose for adults in a low transmission area is 200 mg of artesunate per day simultaneously administered with 500 mg mefloquine per day for 3 days.

In another very preferred embodiment of the invention treating acute uncomplicated *P*. *falciparum* malaria, the effective dose for adults in a high transmission area is 200 mg of artesunate per day simultaneously administered with 250 mg mefloquine per day for 3 days.

For children with an average weight of 25-35 kg the effective amount of treatment is preferably 100 mg of artesunate per day simultaneously administered with 250 mg mefloquine per day for 3 days.

If calculated on the basis of body-weight, the preferred daily dose of artesunate is 4-5 mg/kg/day, the total dose of mefloquine is 20-25 mg/kg, and both doses correspond to the doses as recommended for the monotherapy of the respective drug.

The combination of the present invention is preferably directed to the use in the treatment of malaria caused by *Plasmodium falciparum,* most preferably in the treatment of acute uncomplicated *P*. *falciparum* malaria.

It should be understood that the dosages referred to above are calculated in terms of the drugs per se.

The combination of artesunate and mefloquine according to the present invention may conveniently be used alone or as a pharmaceutical formulation containing pharmaceutically acceptable carriers or diluents and, if desired, additional ingredients such as flavourings, binders, excipients and the like.

Pharmaceutical formulations include those suitable for oral, topical (including dermal, buccal and sublingual), rectal and parenteral (including subcutaneous, intradermal, intramuscular and intravenous) administration as well as administration by naso-gastric tube. The formulation may be prepared by any of the methods well known in the art of pharmacy.

All methods include the step of bringing into association the active ingredients with liquid carriers or finely divided solid carriers or both and then, if necessary, shaping the product into the desired formulation. Pharmaceutical formulations suitable for oral administration, wherein the carrier is a solid, are in general presented as unit dose formulations such as tablets, powders, capsules, sachets and the like.

The combination of the present invention is administered preferably orally, most preferably as tablets. A tablet may be made by compression or moulding, optionally with one or more accessory ingredients. Compressed tablets may be prepared by compressing in a suitable machine the active compounds in a free-flowing form such as a powder or granules, optionally mixed with a binder, lubricant, inert diluent, lubrication agent, surface-active agent or dispersing agent. Moulded tablets may be made by moulding an inert liquid diluent. Tablets may optionally be coated and, if uncoated, may optionally be scored.

For example, tablets containing various disintegrants such as starch, alginic acid and certain complex silicates, together with binding agents such as polyvinylpyrrolidone, sucrose, gelatin and acacia. Additionally, lubrication agents such as magnesium stearate, sodium lauryl sulfate and talc are often useful for tabletting purposes.

Solid compostions of a similar type may also be employed as fillers in soft and hard gelatin capsules, preferred materials therefore include lactose or milk sugar and high molecular weight polyethylene glycols. For oral administration such formulation could be based on a suitable refined edible oil such as sunflower oil, corn oil, and the like.
Sachets are analogous to tablets wherein the active ingredients together with any accessory ingredient(s) are sealed in a rice paper envelope.

Blister packs are well known forms in the art of pharmacy. Tin Shwe et al evaluate in a study the influence of a 5-day blister pack on patients' compliance. A complex dosage regimen was packed and distributed together containing artesunate, mefloquine, quinine and chloroquinine. Tin Shwe et al report improved compliance of the patients as the blister packs ensured that the drugs were sold and taken together. (WHO Bulletin OMS. Vol 76, Suppl. 1, 1998, p. 35-41).

In the present invention a single blister pack has been designed in such a way that the intake of the combined daily dose of drugs is unmistakable and distinctive. It is, therefore, a further preferred embodiment of the present invention that the effective amounts of artesunate and mefloquine are pre-packed in blisters of a single blister pack which is divided into daily units, preferably so that the daily units are distinguished by different colours, conveniently on the back of the blister pack. For example, Day 1 is red, Day 2 is blue and Day 3 is green.

The present blister pack is very easy to handle. The different colours of the units of the blister pack mark the daily intake in a simple and striking way so that even children, patients with poor eyesight, or patients who are illiterate can easily handle the daily dose of intake without failure. Compliance of the patient, which is a crucial factor in the successful treatment of malaria, is ensured by the present blister pack.

The blister pack is illustrated by the drawings without being limited to them.

The antimalarial agents artesunate and mefloquine are known compounds. Artesunate is a derivative of artemisinin and corresponds to the compound dihydroartemisinin hemisuccinate. It has been disclosed, for example, in US Patent No. 6,306,896 and in various reports, e.g. WHO Report of the Scientific Working Group on the Chemotherapy of Malaria, PDR/Chemal 3rd Review, 85.3, Geneva, June 3-5, 1985, and references contained therein. Mefloquine as compound was first described by Ohnmacht et al (J. Med. Chem. 1971,14:926). Its antimalarial activity was disclosed for example by Basco et al (Br. J. Clin. Pharmac., 1992,33:517-520).

The present invention is illustrated by the following examples. It should be, however, understood that the invention is not limited to the specific details of the examples.

### Example 1

### Preparation of the artesunate and mefloquine formulations

Lactabs (film-coated tablets) having a composition as listed below were prepared using conventional techniques:
a) Artesunate Lactab

| **INGREDIENT** | **mg/tablet** |
|---|---|
| Artesunate | 200.0 |
| Microcristalline cellulose | 170.0 |
| Lactose monohydrate | 135.0 |
| Croscarmellose sodium | 12.5 |
| Hydroxypropyl methyl cellulose (hypromellose) | 10.0 |
| Talc | 7.0 |
| Magnesium stearate | 3.0 |
| Maize starch | 9.5 |
| Colloidal silicon dioxide | 3.0 |
| Polyethylene glycol 6000 (macrogol) | 10.0 |

| **FILM COAT** | |
|---|---|
| Hydroxypropyl methyl cellulose (hypromellose) | 10.0 |
| Titanium dioxide | 1.4 |
| Polyethylene glycol 6000 (macrogol) | 0.6 |

b) Mefloquine Lactab

Each mefloquine lactab contains 250 mg mefloquine base in the form of 275 mg mefloquine hydrochloride.

| **INGREDIENT** | **mg/tablet** |
|---|---|
| Mefloquine hydrochloride | 275.00 |
| Microcristalline cellulose | 89.00 |
| Sodium lauryl sulfate | 10.00 |
| Sodium carboxymethylcellulose | 30.00 |
| Lactose 200 mesh | 24.50 |
| Lactose DC | 57.50 |
| Hydroxypropyl methyl cellulose | 10.00 |
| Magnesium stearate | 3.00 |
| Pregelatinised starch | 6.00 |
| Colloidal silicone dioxide | 7.50 |
| Polyethylene glycol 6000 (macrogol) | 7.50 |

| **FILM COAT** | |
|---|---|
| Talc | 3.00 |
| Titanium dioxide | 4.00 |
| Hydroxypropyl methyl cellulose | 3.80 |
| Polyethylene glycol | 0.67 |

### Example 2

### Clinical study in a high transmission area.

A randomised, double-blind, parallel group, comparative study in 104 patients (adults and children) with acute, uncomplicated *Plasmodium falciparum* malaria was performed in 3 centres in Africa (Benin, Cameroon and Ivory Coast) with the aim to evaluate the tolerability, safety and efficacy of artesunate-mefloquine dosage regimen which was co-administered once daily for 3 days in blister packs as described above.

Patients (adults and children with body-weight between 30 and 55 kg) were randomised to receive accordingly in:
- Group A: artesunate 200 mg/day and mefloquine 250 mg/day simultaneously once daily for 3 days (according to the invention) ;
- Group B: artesunate 200 mg/day and mefloquine 750 mg total dose sequentially (no mefloquine dose on the 1^{st} day, 250 mg on the 2^{nd} day and 500 mg on the 3^{rd} day) once daily for 3 days (reference group).

Patients were followed up for 28 days, and clinical and parasitologic outcomes were assessed. Primary efficacy endpoint was the 14-day cure rate, i.e. proportion of patients with clearance of asexual parasiemia within 7 days of initiation of study treatment, without subsequent recrudescence within 14 days.

### Results:

| **Results** | **Group A** | **Group B** |
|---|---|---|
| Cure rate after 14 days | 100 % | 98 % |
| Cure rate after 28 days | 100 % | 98 % |
| Early vomiting (within 30 minutes) | 1.9 % | 9.6 % |
| Overall vomiting | 3.8 % | 19.2 % |
| Parasite clearance time | 45 hours | 48 hours |
| Fever clearance time | 32 hours | 26 hours |

The result show the high efficacy rate of both artesunate-mefloquine combination regimen, wherein the main parameters are comparable, such as cure rates, parasite and fever clearance times. Of considerable importance is the similar cure rate at day 14 and 28, which show that no recrudescence of malaria infection occured during a 28-day follow-up periode in a high transmission malaria area.

Also remarkable is the low incidence rate of early vomiting and overall vomiting in Group A, where both drugs were co-administered from the first day on. Actually, the opposite had to be expected based on the prior art. Contrary to this, the results show that splitting the total mefloquine dose in three equal daily doses offer a real benefit in terms of compliance with improved tolerability.

### Example 3

### Clinical study in a low transmission area.

Similar to Example 2, a randomised, double-blind, parallel group, comparative single-centre study in 204 patients (adults and children) was carried out in Asia (Thailand).

Patients were randomised to receive in:
- Group A: artesunate 200 mg/day and mefloquine 500 mg/day simultaneously once daily for 3 days (according to the invention)
- Group B: artesunate 200 mg/day and mefloquine 1500 mg total dose sequentially (no mefloquine dose on the 1^{st} day, 750 mg on the 2^{nd} day and 750 mg on the 3^{rd} day) once daily for 3 days (reference group).

Patients (children with body-weight 25-50 kg and adults) received the respective amount of drugs corresponding to their body-weight.

Primary efficacy endpoint was the 28-day cure rate.

### Results:

| **Results** | **Group A** | **Group B** |
|---|---|---|
| Cure rate after 28 days | 100 % | 99 % |
| Overall vomiting | 2.9 % | 2.0 % |
| Parasite clearance time | 44 hours | 48 hours |
| Fever clearance time | 34 hours | 30 hours |
| CNS side effects | 4.9 % | 8.8 % |

The result show the high efficacy rate of both artesunate-mefloquine combination regimen, wherein the main parameters are comparable, such as cure rates, parasite and fever clearance times. Tolerablity was not compromised by administering mefloquine simultaneously with artesunate on Day 1, as the rates for overall vomiting indicate.

While both dose regimens give very good treatment results, the CNS side effects of Group A are favourably lower.

Examples 2 and 3 prove that the present artesunate-mefloquine combination in the treatment of malaria, if co-administered simultaneously once daily, is equal in efficacy and safety to currently used dose regimens, and in some respect of tolerability is even superior.

The present method of treatment is, therefore, very suitable for co-administration in a single blister pack of 3 equal daily dose units in order to improve patients' compliance in an effective way.

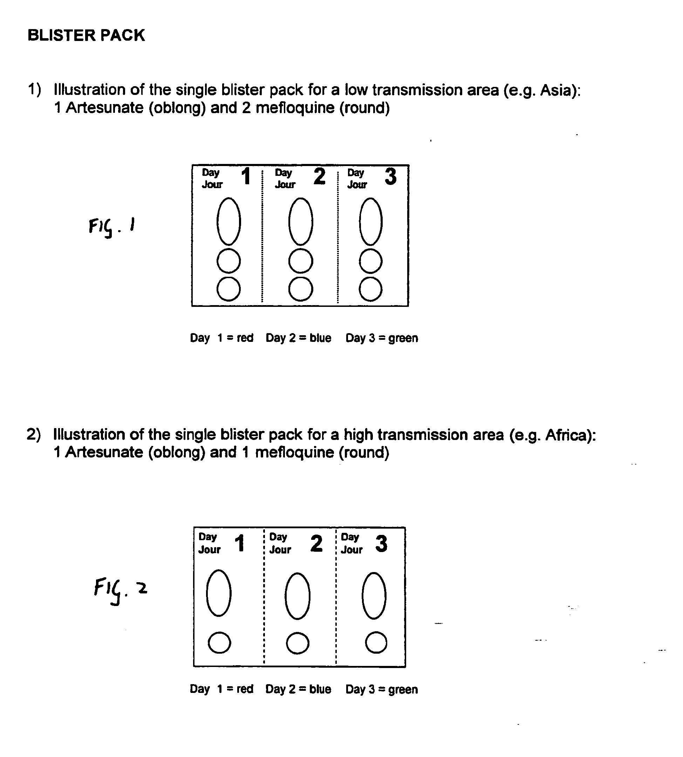

## Claims

1. A pharmaceutical combination for the treatment of malaria in a 3-day course comprising an effective amount of artesunate and of mefloquine wherein the dosage of artesunate and mefloquine is each day the same.

2. A combination according to claim 1 wherein the amount of artesunate is 20-700mg per day, and the amount of mefloquine is 100-900mg per day.

3. A combination according to claim 1 wherein the amount of artesunate is 50-550mg per day, and the amount of mefloquine is 150-750mg per day.

4. A combination according to claim 1 wherein the amount of artesunate is 200mg per day, and the amount of mefloquine is 250-500mg per day.

5. A combination according to claim 4 wherein the amount of artesunate is 200mg per day, and the amount of mefloquine is 250mg per day.

6. A combination according to claim 4 wherein the amount of artesunate is 200mg per day, and the amount of mefloquine is 500mg per day.

7. A combination according to claim 1 wherein the effective amount of artesunate and of mefloquine are pre-packed as separate dosage forms in blisters of a single blister pack which is divided into daily units.

8. A combination according to claim 7 wherein the the daily units are distinguished by different colors.

9. A combination according to any of claims 1 to 8 for use in the treatment of malaria caused by *Plasmodium falciparum*.

10. A blister pack which comprises the effective amount of the combination according to claim 1 pre-packed as distinguishable daily units.

11. A blister pack according to claim 10 wherein the daily units are distinguished by different colors.

12. A blister pack according to claims 10 and 11 wherein the colors for distinction are on the back of the blister.

13. A combination according to claims 5, 10, 11 and 12 which consists of three doses of two tablets, one artesunate 200mg and one mefloquine 250mg, to be taken simultaneously.

14. A combination according to claims 6, 10, 11 and 12 which consists of three doses of three tablets, one artesunate 200mg and two mefloquine 250mg, to be taken simultaneously.

15. Use of an effective amount of artesunate and of mefloquine in the manufacture of a medicament for the treatment of malaria in a 3-day course wherein the dosage of artesunate and of mefloquine is each day the same.

16. Use of an effective amount of artesunate and of mefloquine in the manufacture of a medicament for the treatment of malaria in a 3-day course according to claim 15 wherein the combination of artesunate and of mefloquine is administered once a day simultaneously.

17. Use of an effective amount of artesunate and of mefloquine in the manufacture of a medicament for the treatment of malaria in a 3-day course according to claims 15 or 16 wherein the combination is administered orally, preferably as tablets.

18. Use of an effective amount of artesunate and of mefloquine in the manufacture of a medicament for the treatment of malaria in a 3-day course according to claim 15 wherein the malaria is caused by *Plasmodium falciparum*.

## Patentansprüche

1. Pharmazeutische Zusammensetzung zur Behandlung von Malaria in einer 3-tägigen Verabreichung umfassend eine wirksame Menge von Artesunat und Mefloquin, wobei die Dosierung von Artesunat und Mefloquin jeden Tag gleich ist.

2. Zusammensetzung nach Anspruch 1, wobei die Menge Artesunat täglich 20-700 mg und die Menge Mefloquin täglich 100-900 mg beträgt.

3. Zusammensetzung nach Anspruch 1, wobei die Menge Artesunat täglich 50-550 mg und die Menge Mefloquin täglich 150-750 mg beträgt.

4. Zusammensetzung nach Anspruch 1, wobei die Menge Artesunat täglich 200 mg und die Menge Mefloquin täglich 250-500 mg beträgt.

5. Zusammensetzung nach Anspruch 4, wobei die Menge Artesunat täglich 200 mg und die Menge Mefloquin täglich 250 mg beträgt.

6. Zusammensetzung nach Anspruch 4, wobei die Menge Artesunat täglich 200 mg und die Menge Mefloquin täglich 500 mg beträgt.

7. Zusammensetzung nach Anspruch 1, wobei die wirksame Menge Artesunat und Mefloquin in separaten Dosierungsformen in Blister einer einzelnen Blisterpackung, welche in Tageseinheiten geteilt ist, vorverpackt ist.

8. Zusammensetzung nach Anspruch 7, wobei die Tageseinheiten sich durch verschiedene Farben unterscheiden.

9. Zusammensetzung nach einem der Ansprüche 1 bis 8 für die Verwendung zur Behandlung von Malaria, welche durch *Plasmodium falciparum* verursacht wird.

10. Blisterpackung, welche die wirksame Menge der Zusammensetzung nach Anspruch 1 umfasst, welche in unterscheidbare Tageseinheiten vorverpackt ist.

11. Blisterpackung nach Anspruch 10, in welcher die Tageseinheiten sich durch verschiedene Farben unterscheiden.

12. Blisterpackung nach den Ansprüchen 10 und 11, in welcher sich die Farben zur Unterscheidung auf der Rückseite des Blisters befinden.

13. Zusammensetzung nach den Ansprüchen 5, 10, 11 und 12, welche aus drei Dosen von zwei Tabletten besteht, eine von Artesunat 200 mg und eine von Mefloquin 250 mg, welche gleichzeitig eingenommen werden.

14. Zusammensetzung nach den Ansprüchen 6, 10, 11 und 12, welche aus drei Dosen von drei Tabletten besteht, eine von Artesunat 200 mg und zwei von Mefloquin 250 mg, welche gleichzeitig eingenommen werden.

15. Verwendung einer wirksamen Menge von Artesunat und Mefloquin in der Herstellung eines Medikaments zur Behandlung von Malaria in einer 3-tägigen Verabreichung, wobei die Dosierung von Artesunat und Mefloquin jeden Tag gleich ist.

16. Verwendung einer wirksamen Menge von Artesunat und Mefloquin in der Herstellung eines Medikaments zur Behandlung von Malaria in einer 3-tägigen Verabreichung nach Anspruch 15, wobei die Zusammensetzung von Artesunat und Mefloquin einmal täglich gleichzeitig verabreicht wird.

17. Verwendung einer wirksamen Menge von Artesunat und Mefloquin in der Herstellung eines Medikaments zur Behandlung von Malaria in einer 3-tägigen Verabreichung nach Anspruch 15 oder 16, wobei die Zusammensetzung oral, vorzugsweise in Form von Tabletten, verabreicht wird.

18. Verwendung einer wirksamen Menge von Artesunat und Mefloquin in der Herstellung eines Medikaments zur Behandlung von Malaria in einer 3-tägigen Verabreichung nach Anspruch 15, wobei die Malaria durch *Plasmodium falciparum* verursacht wird.

## Revendications

1. Formulation pharmaceutique pour le traitement du paludisme en 3 jours comprenant une quantité efficace d'artesunate et de mefloquine, où le dosage d'artesunate et de mefloquine est tous les jours pareil.

2. Formulation selon la revendication 1, où la quantité d'artesunate est de 20-700 mg par jour, et la quantité de mefloquine est de 100-900 mg par jour.

3. Formulation selon la revendication 1, où la quantité d'artesunate est de 50-550 mg par jour, et la quantité de mefloquine est de 150-750 mg par jour.

4. Formulation selon la revendication 1, où la quantité d'artesunate est de 200 mg par jour, et la quantité de mefloquine est de 250-500 mg par jour.

5. Formulation selon la revendication 4, où la quantité d'artesunate est de 200 mg par jour, et la quantité de mefloquine est de 250 mg par jour.

6. Formulation selon la revendication 4, où la quantité d'artesunate est de 200 mg par jour, et la quantité de mefloquine est de 500 mg par jour.

7. Formulation selon la revendication 1, où la quantité efficace d'artesunate et de mefloquine est conditionnée en des formes de dosage séparées dans des plaquettes thermoformées d'un seul emballage de plaquettes thermoformées qui est partagé en unités journalières.

8. Formulation selon la revendication 7, où les unités journalières se distinguent par différentes couleurs.

9. Formulation selon l'une quelconque des revendications 1 à 8 pour l'utilisation dans le traitement du paludisme causé par le *plasmodium falciparum*.

10. Emballage de plaquettes thermoformées qui comprend la quantité efficace de la formulation selon la revendication 1 conditionnée sous forme d'unités journalières distinguables.

11. Emballage de plaquettes thermoformées selon la revendication 10, où les unités journalières se distinguent par différentes couleurs.

12. Emballage de plaquettes thermoformées selon les revendications 10 et 11, où les couleurs pour la distinction se trouvent sur l'arrière de la plaquette thermoformée.

13. Formulation selon les revendications 5, 10, 11 et 12, qui consiste en trois doses de deux comprimés, une d'artesunate 200 mg et une de mefloquine 250 mg, à prendre simultanément.

14. Formulation selon les revendications 6, 10, 11 et 12, qui consiste en trois doses de trois comprimés, une d'artesunate 200 mg et deux de mefloquine 250 mg, à prendre simultanément.

15. Utilisation d'une quantité efficace d'artesunate et de mefloquine dans la fabrication d'un médicament pour le traitement du paludisme en trois jours où le dosage d'artesunate et de mefloquine est tous les jours pareil.

16. Utilisation d'une quantité efficace d'artesunate et de mefloquine dans la fabrication d'un médicament pour le traitement du paludisme en trois jours selon la revendication 15 où la formulation d'artesunate et de mefloquine est administrée une fois par jour simultanément.

17. Utilisation d'une quantité efficace d'artesunate et de mefloquine dans la fabrication d'un médicament pour le traitement du paludisme en trois jours selon les revendications 15 ou 16 où la formulation est administrée oralement, de préférence sous forme de comprimés.

18. Utilisation d'une quantité efficace d'artesunate et de mefloquine dans la fabrication d'un médicament pour le traitement du paludisme en trois jours selon la revendication 15 où le paludisme est causé par le *plasmodium falciparum*.
